# EUROPEAN PATENT SPECIFICATION

(11) **EP 4 466 954 B1**
(45) Date of publication and mention of the grant of the patent: **04.03.2026**
(21) Application number: 23701286.9
(22) Date of filing: 17.01.2023
(51) Int. Cl.: H05H 1/00, H01J 37/32

(54) **PLASMA DISCHARGE MONITORING METHOD**
VERFAHREN ZUR ÜBERWACHUNG EINER PLASMAENTLADUNG
MÉTHODE DE SURVEILLANCE D'UNE DÉCHARGE PLASMA

(30) Priority: 18.01.2022 GB 202200530
(43) Date of publication of application: 27.11.2024
(73) Proprietor: UCL Business Ltd, London Greater London WC1E 6BT (GB)
(72) Inventor: SENER, M. Emre, London Greater London NW3 4EE (GB); CARUANA, Daren J., London Greater London SE9 3PY (GB)
(74) Representative: Mewburn Ellis LLP
(86) International application number: PCT/EP2023/051018
(87) International publication number: WO 2023/139066

(56) References cited:
- EP-B1- 3 602 602
- WO-A1-2021/047761
- WO-A2-2004/006298
- GB-A- 2 459 858

## Description

### Field of the Invention

The present invention relates to methods and systems for monitoring a characteristic of a surface while impinging a plasma discharge on the surface.

### Background

Atmospheric pressure plasma discharges are widely used in industry and medical settings for a diverse range of applications including surface treatment, surface modification, and material deposition applications. An emerging method of generating an atmospheric pressure plasma discharge is via pulsed radio frequency (RF) excitation, wherein pulse width modulated bursts of RF energy are used to generate a plasma discharge. In such applications, the pulsed nature of the plasma discharge can be used to prevent excess thermalisation and damage to surfaces in contact with the discharge.

During plasma processing (e.g. the plasma deposition of films), control of the plasma discharge intensity is critical to prevent formation of arcs and other high energy events which could result in surface damage due to excess power, as well as plasma ignition failures. Similarly, using a too low discharge power can lead to deposit or process failures. In medical settings, fine control over the discharge intensity is critical to prevent inadvertent tissue damage whilst still ensuring treatment efficacy.

Another problem is that changes in substrate characteristics can have rapid and unpredictable effects on RF plasma parameters and characteristics. This is because the power coupled to a surface by a plasma discharge emitted from a plasma nozzle (also known as a plasma jet) is highly dependent on the electrical characteristics of the surface itself. As such, in situations where conductive or insulating layers are deposited onto surfaces, the surface electrical properties are modified which can have an effect on the plasma discharge parameters. Similarly, in medical settings, plasma treatment of electrically dissimilar tissues results in unpredictable behaviour of the plasma discharge.

Existing methods for controlling plasma discharge involve limiting the power delivered by the electrical plasma apparatus. While this may be effective in preventing arcs and burns, these methods do not establish optimum discharge characteristics. These methods are also incapable of providing any feedback during the plasma process.

Other methods rely on direct (time domain) sampling or probing of plasma currents and voltages. However, these methods can be difficult and costly to implement due to bandwidth requirements. Furthermore, due to the high impedance nature of the plasma discharge, where significantly high voltages may be present, specialized equipment is often required to implement these conventional probing techniques. WO 2004/006298 A2 discloses an antenna-based RF sensor for remotely and non-invasively sensing and analyzing parameters of a plasma processing tool.

The present invention has been devised in light of the above considerations.

### Summary of the Invention

The invention is defined by the appended claims.

According to a first aspect of the invention, there is provided a method for monitoring a characteristic of a surface while an atmospheric-pressure dual-frequency plasma discharge is impinging on the surface, the method comprising:
receiving a signal emitted from the plasma discharge impinging on the surface;
performing a frequency domain analysis of the signal; and
generating an output signal indicative of a characteristic of the surface based on the frequency domain analysis.

By using a plasma discharge that is at atmospheric pressure and is dual-frequency driven, the characteristics of the surface, rather than the bulk of the plasma discharge itself, can be monitored non-invasively.

Although not wishing to be bound by theory, the inventors have found that the surface characteristics can be probed using this method for the following reasons.

Atmospheric pressure plasma discharges are highly non-linear loads, and they therefore generate significant harmonic distortion leading to emission of electromagnetic radiation at integer multiples of the driving waveform frequency. When using plasma discharges emitted from dual-frequency (e.g. pulsed) driven plasma nozzles, different amounts of non-linearity may be present at different times during the plasma discharge generation. For example, in pulsed plasma discharges, the distortion resulting from the non-linearity of the atmospheric pressure plasma discharge is only present during the actual plasma generator pulse "on-time", where plasma discharge is present. This period where the actual plasma discharge is present may differ from the "on-time" of the ideal modulating waveform, and differing amounts of non-linearity may be present at different times during the plasma discharge generation. This leads to highly distorted modulating waveforms at harmonics of the fundamental driving frequency.

The increasing density of the plasma discharge between the plasma nozzle (e.g. driver electrode) and the surface upon which the plasma discharge is impinged effectively serves as a resistance load, decreasing the impedance of the plasma discharge, compared to in the absence of the surface. By coupling to a surface with finite impedance, the plasma load in the circuit is increased, causing significant differences in the modulation envelope that depend entirely on the electrical characteristics of the surface upon which the plasma discharge impinges. This effectively generates a signature distortion of the modulating waveform that is dependent on the plasma-facing surface, and this distortion can be analysed in the frequency domain to extract information about the surface itself.

As a result of being able to monitor the surface characteristics non-invasively, it is possible to use this method alongside existing plasma apparatus equipment, and specialized plasma equipment is not required. Thus, implementation of the method is simpler than conventional methods. Furthermore, as the method is non-invasive, there is no requirement for optical or electrical access to the plasma discharge.

Optional features of the invention will now be set out. These are applicable singly or in any combination with any aspect of the present disclosure.

As used herein, the term plasma discharge and plasma plume are interchangeable. A plasma discharge may be understood as referring to ionised gas discharged from a plasma nozzle (also known as a plasma jet) driven by a driving signal.

Preferably, the plasma discharge is a radio frequency (RF) plasma discharge. The plasma discharge may be emitted from a plasma nozzle driven by a driving signal (e.g. RF carrier fundamental) with a frequency between 20 kHz and 300 GHz. More preferably, the plasma discharge may be emitted from a plasma nozzle driven by a driving signal with a frequency between 500 kHz and 1 GHz.

The dual-frequency plasma discharge may be amplitude modulated (AM). For example, the plasma discharge may be a pulsed plasma discharge, e.g. equivalent to a plasma discharge with an 100% AM modulation depth. The AM plasma discharge may have a different modulation depth (e.g. in the range from 20%-100%, more preferably 40%-100%, more preferably 60%-100%, more preferably 80%-100%, more preferably 90%-100%).

The plasma discharge may be a hot plasma discharge, or a cold plasma discharge.

The signal emitted from the plasma discharge impinging on the surface may be captured non-invasively. For example, the signal may be captured by a probe. The probe may be a non-contact probe. The probe may be a non-loading and/or non-reactive probe (e.g. a probe that can capture the signal without having to put an electrical load on the plasma apparatus and/or which is not electrically reactive).

The signal may be a near-field signal. For example, the signal may be captured/sampled in the near-field region, e.g. the reactive near-field and/or the radiative Fresnel region. Alternatively, the signal may be a far-field signal, such that the signal may be captured/sampled in the far-field region. The signal may be captured in the far-field if it is sensitive enough to detect one or more higher harmonics of a driving signal present in the signal. In particular, the signal may be sampled at any distance as long as the probe sampling the signal is sensitive enough to detect one or more higher harmonics of the driving signal present in the signal.

It is to be understood that a harmonic is a signal with a frequency that is a positive integer multiple of the frequency of the driving signal. The first harmonic is the driving signal. A higher harmonic has a frequency of at least 2x the frequency of the driving signal.

As used herein, a near-field signal may be a signal evidenced by a 1/r³ intensity drop, where r is the distance from the signal source. A far-field signal may be a signal evidence by a 1/r² intensity, where r is the distance from the signal source.

In atmospheric pressure discharges, the characteristic plasma dimensions may be shorter than the wavelength of the driving waveform (e.g. the driving signal, λ_{f}). For these discharges, the near-field region may be understood as the region that lies within λ_{f} from the plasma discharge. The reactive near-field region may be understood as the region within λ_{f}/2π of the plasma discharge. The radiative near-field region may be understood as the region lying within a distance d away from the plasma discharge, wherein λ_{f}/2π < *d <* λ_{f}. Beyond the near field region, the far field radiation dominates.

The methods disclosed herein may be used in the reactive near-field region (e.g. such that the probe captures the signal in the reactive near-field region) where an appreciable stray impedance may be imposed onto the plasma discharge, perturbing it. The methods disclosed herein may also be used in the radiative near-field and far field regions, where passive sampling does not perturb the plasma discharge. In the near-field region, it is preferred that the signal is captured at a fixed position with respect to the plasma discharge as the radiation pattern is non-uniform at these distances. In contrast, with far-field sampling, sampling at a fixed position is less important.

Optionally, the signal may be captured using an antenna. The antenna may be an E-field antenna. The antenna may be a near-field, or far-field antenna. The antenna may be a random-wire, dipole, or directional antenna, for example. The antenna may be optimized for Very High Frequency (VHF), e.g. 30-300 MHz, signals, or Ultra High Frequency (UHF), e.g. 300 MHz - 3 GHz, signals.

Alternatively, the signal may be received by a capacitive or inductive probe e.g. by direct capacitive/inductive coupling to the driving wire of a nozzle generating the plasma (e.g. to sample the signal in electrode wires of the plasma nozzle). In these examples, the signal is sampled in the reactive near-field region.

The method may comprise impinging the plasma discharge on the surface.

The method may comprise adjusting the power delivered to the plasma discharge by a generator, based on the output signal. The generator may be an radio frequency (RF) generator, for example,

In particular, the voltage and/or current applied by the driving signal may be adjusted based on the output signal, in order to adjust the power delivered to the plasma discharge by the generator. Alternatively/additionally, the method may comprise adjusting the frequency of the driving signal applied by the generator to the plasma discharge, based on the output signal.

Accordingly, the output signal indicative of a characteristic of the surface upon which the plasma discharge impinges may be fed back in a control loop *in situ* to maintain a stable plasma dissipated power despite changes in the characteristic of the surface and/or other changing environmental conditions. As such, the method may be used as a process control technique.

In particular, the power delivered by the generator may be adjusted such that the power dissipated to the surface may be maintained as approximately constant.

The method may comprise generating a spatial and/or temporal map of the characteristic of the surface by recording the output signal as a function of time and/or spatial position.

Accordingly, it is possible to record changes in the characteristic of the surface over time and/or map changes in the characteristic over the surface. In this way, conductive inclusions or non-uniform sections that may be present underneath a plasma exposed surface may be detected and mapped. As such, the method may be used as a surface characterisation method. The spatial resolution may be limited by the size of the plasma discharge, extending down to the micrometre range.

In order to generate a spatial map of the characteristic of the surface, the surface may be moved relative to the (e.g. stationary) plasma nozzle (e.g. so that the plasma discharge impinges on a different portion of the surface as the surface moved relative to the plasma nozzle). The surface may be moved in 3D space (e.g. X-Y-Z directions).

Alternatively, the plasma nozzle may be moved relative to the (e.g. stationary) surface. The plasma nozzle may be moved in 3D space (e.g. X-Y-Z directions).

In some examples, the method may further comprise outputting the spatial/temporal map of the characteristic of the surface for display, e.g. on a screen. The spatial/temporal map may be output to a remote device (e.g. via a wired or wireless connection).

The plasma discharge may be impinging on the surface to deposit, modify or remove a layer thereon. The surface may be a surface of a substrate, for example. The layer may have different electrical properties to the surface/substrate.

By using the method disclosed herein in conjunction with plasma deposition techniques, it can be ensured that the deposited layers are uniform, or in the situation of conductive material deposition, that the deposited layers are end-to-end conductive.

When such plasma discharge deposition/removal/modification techniques are used in conjunction with the process control loop described above, reliable, and repeatable deposition/removal/modification of the layer is possible, under fixed dissipated plasma power.

Optionally, the characteristic of the surface is surface impedance. As used herein, the term surface impedance is to be understood as meaning the complex surface impedance at a chosen harmonic frequency of a driving signal present in the signal. The term "surface" is to be understood as being equivalent to 1-2 skin depths at which currents at the chosen harmonic frequency travel (wherein the skin depth is the distance below the actual surface of a material where the current density has diminished to 1/e of its value at the surface).

As discussed above, the frequency domain analysis of the signal relies on the generation of harmonics in the radio frequency emissions as the plasma discharge impinges on the surface.

In some examples, the frequency domain analysis may comprise generating a power spectral density (e.g. data representing the power spectral density) of the signal by performing a Fourier Transform analysis. The output signal may be generated at least in part by performing a data fitting process to resonant features of the power spectral density. The data fitting process may be fitting a data series or a shape parameter to the resonant features for example.

Preferably, the frequency domain analysis may comprise evaluating resonant features of at least one higher harmonic of a driving signal present in the signal. For example, the frequency domain analysis may comprise evaluating resonant features of one or more of the second to the thirteenth harmonic, more preferably of the second to the tenth harmonic, more preferably the third to the eighth harmonic. For example, the frequency domain analysis may comprise evaluating resonant features of one or more of the fourth, fifth or sixth harmonic.

In some examples, the frequency domain analysis may comprise evaluating a relative intensity of a plurality of higher harmonics of the driving signal present in the signal. For example, the relative intensity of the fourth, sixth and eighth harmonic may be evaluated. It is understood that other combinations of harmonics may also be evaluated together. The harmonics to be evaluated may be chosen based on the sensitivity of the probe/antenna and/or on the method for performing the frequency domain analysis, for example.

By evaluating the higher harmonics, more detailed information relating to the surface characteristics can be observed. This is because the modulation information carried by the weaker, higher harmonics are nearly entirely due to the presence of the plasma discharge impinging on the surface, whereas the modulation information carried by the significantly stronger driving signal (e.g. fundamental RF signal) is due to both displacement currents in plasma circuitry and the bulk of the plasma discharge itself.

The output signal may be generated, at least in part, by a machine learning process. Using a machine learning process may allow for a plurality of higher harmonics to be evaluated together, as described above. The machine learning process may be supervised or unsupervised. Unsupervised learning methods may be employed to differentiate between different features that may be present in the plasma discharge exposed surface, such as electrical conductivity differences or sub-surface faults that may be present in an otherwise conductive (or insulating) surface (e.g. substrate). Supervised learning methods, which may utilize deep neural networks, may be used with pre-collected training data for classification of different types of test surfaces by feeding in spectral features from labelled data and using the pre-trained neural network for classification of the surface characteristic.

For example, in the deposition of conductive materials, the training set may comprise two extreme cases; (i) power spectral densities collected while the plasma discharge is impinging on a conductive surface of a substrate with a desired thickness and with desired electrical properties; and (ii) power spectral densities while the plasma discharge is impinging on a bare non-conductive substrate surface. A machine learning model can then track the process as a conductive layer is deposited on the surface, as the signal emitted from the plasma discharge impinging on the surface changes over time from case (ii) to case (i). The method may comprise filtering the signal to remove high power signals and/or to remove background noise.

The filtering may be performed at the antenna, for example. In particular, the fundamental frequency of the driving signal may be high pass filtered from the signal. Alternatively/additionally, digital signal processing methods may be used to notch filter interference. In some examples, background noise may be detected, e.g. with a second receiver placed remotely from the plasma discharge (or further away from the plasma discharge than the antenna/prove), which may then be subtracted/removed from the signal. The method may comprise discarding phase information of the signal when transformed into the frequency domain. This may reduce the processing load, and thus processing time, for generating the output signal.

In medical applications, the surface may be a tissue surface, e.g. surface of human skin. The surface may be a human or animal tissue surface.

In some applications, by impinging on the surface, the plasma discharge may be used in a process for plasma activating and/or cleaning the surface.

The method may be computer-implemented. For example, the method may be performed by one or more processors, e.g. one or more computing devices or servers.

The method may comprise the step of capturing (or sampling), using an antenna, the signal emitted from the plasma discharge impinging on the surface.

According to a second aspect of the invention there is provided a system for performing the method of the first aspect. Accordingly, there is provided a system for monitoring a characteristic of a surface while an atmospheric-pressure dual-frequency plasma discharge is impinging on the surface, the system comprising a plasma apparatus, the plasma apparatus comprising a radio frequency generator and an atmospheric pressure plasma nozzle configured to be driven by the radio frequency generator. The plasma apparatus is configured to generate the plasma discharge, which is discharged from the plasma nozzle. The system further comprises one or more processors configured to:
receive a signal emitted from the plasma discharge impinging on the surface, wherein the signal is captured using an antenna;
perform a frequency domain analysis of the signal; and
generate an output signal indicative of a characteristic of the surface based on the frequency domain analysis.

The system may comprise a memory configured to store instructions which, when executed by the one or more processors, cause the one or more processors to perform the method of the first aspect.

The system may further comprise an antenna. The antenna may be configured to capture the signal emitted from the plasma discharge impinging on the surface. The signal may be received by the one or more processors from the antenna, wherein the one or more processors then perform the frequency domain analysis and generate the output signal indicative of a characteristic of the surface.

According to a third aspect, there is provided use of the system of the second aspect.

According to a fourth aspect, there is provided a non-transitory computer-readable storage medium containing machine executable instructions which, when executed on a processor, cause the processor to perform the method of the first aspect.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
Figure 1 is a schematic diagram of a system for performing a method for monitoring a characteristic of a surface;
Figure 2 is a flowchart illustrating a method for monitoring a characteristic of a surface;
Figure 3 is a schematic diagram of an example system for performing a method for monitoring a characteristic of a surface;
Figure 4 shows an example test standard and physical sample of a reinforced circuit board that can be used with the example system of Figure 3;
Figure 5 shows graphs of current and voltage waveforms as obtained experimentally and through simulations using the example system of Figure 3;
Figure 6A shows circuit diagrams associated with the example system of Figure 3;
Figure 6B includes plots of pulse envelopes during activation of the example system of Figure 3;
Figure 7A shows pairwise plots 500 of principal component loadings for each datapoint in a map in the analysis of an example signal;
Figure 7B shows a spatial map of the surface impedance generated by the example system of Figure 3;
Figure 8 shows metallic tracks deposited on a substrate;
Figure 9A is a decision tree of the logic for controlling the power supplied to the plasma discharge in the method of Figure 2;
Figure 9B is a plot of the power signal used to control RF power amplified levels during a plasma deposition process in the method of Figure 2; and
Figure 10 is a waterfall plot of a plasma discharge periodically encountering a grounded surface.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

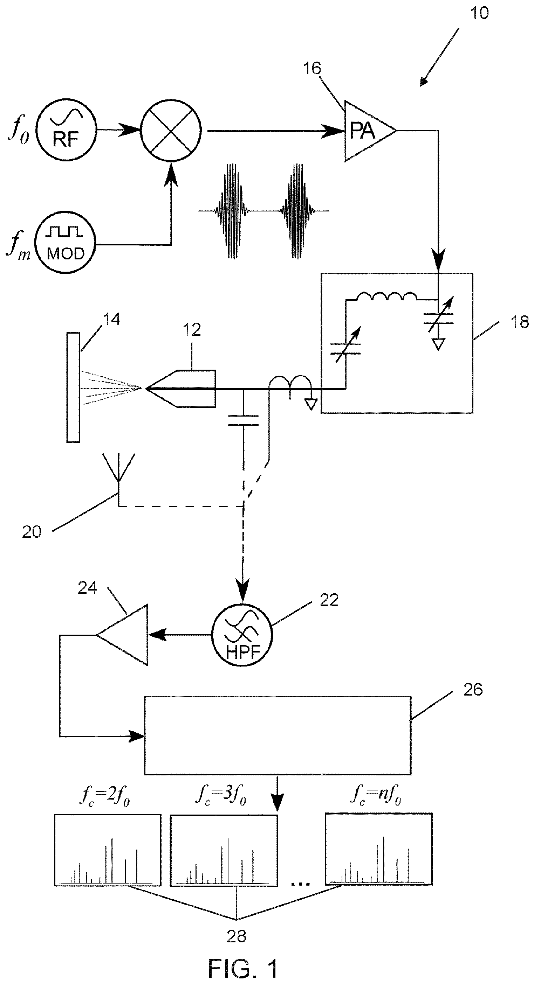

Figure 1 is a schematic diagram of a system 10 for performing a method for monitoring a characteristic of a surface. The system 10 comprises a nozzle 12 for generating an atmospheric pressure dual-frequency plasma discharge which impinges on a surface 14. The nozzle 12 is driven by a pulsed RF waveform formed from an RF carrier signal *f₀* that is modulated with a pulse frequency *fₘ.* The signal is amplified at an amplifier 16, and then impedance matched to the plasma load via a RF matching network 18.

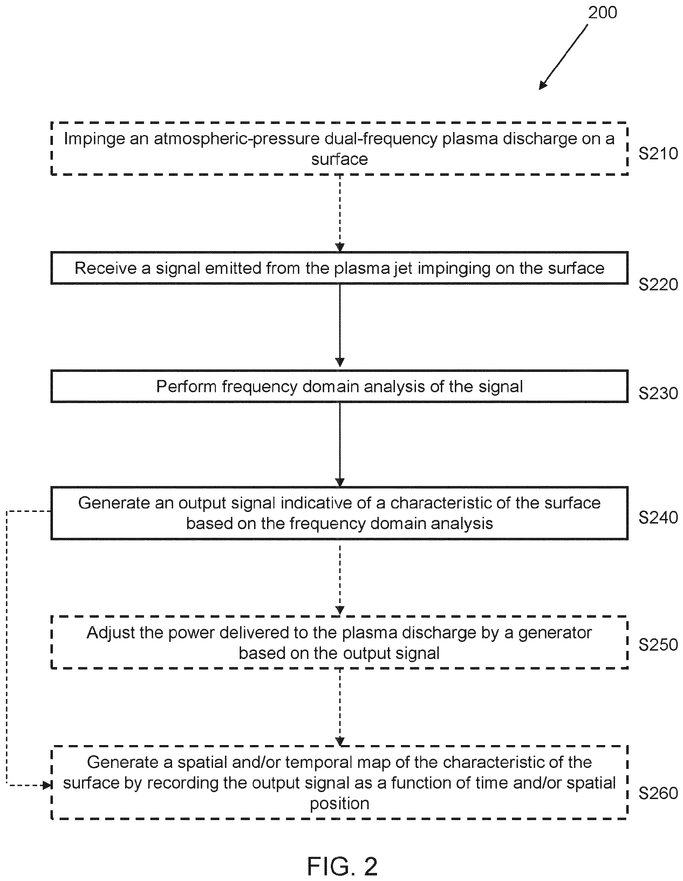

An example method 200 for monitoring a characteristic of a surface is set out in Figure 2. The method 200 may be performed by the system 10 shown in Figure 1.

At S210 of method 200, nozzle 12 generates an atmospheric-pressure, dual-frequency plasma discharge which is controlled to impinge on surface 14.

The plasma discharge may impinge on the surface in order to deposit, modify or remove a layer on the surface, for activating or for cleaning the surface, or in some examples can be used in medical applications.

As the plasma discharge is sustained by the nozzle 12, RF energy is emitted from the plasma discharge which can be sampled. In particular, at S220 of Figure 2, a signal emitted from the plasma discharge is sampled and received.

In the example system 10 shown in Figure 1, the RF energy is sampled with an antenna 20. In other examples, the RF energy may be sampled using another type of probe, or by direct sampling of the electrode wires in the portion of the circuit between the match network 18 and the plasma nozzle 12.

During the plasma generation process, non-linearities in the plasma load results in the generation of harmonics at integer values of the RF carrier fundamental signal *f_{0.}* The pulse modulation information is also present, with sidebands *nf₀,* where n is any positive integer, generated around a central frequency of the RF carrier fundamental signal, *f₀*. This results in a power spectrum in the frequency domain that is composed of sidebands *nf₀* of varying amplitude, evenly separated by *fₘ* around the centre frequency *f₀*.

The signal captured by the antenna is then analysed.

As mentioned above, pulse modulation information is present in the signal. The modulation information carried by the weaker (higher) harmonics is substantially due to the presence of the electrical plasma discharge whereas the modulation information carried by the significantly stronger fundamental RF signal is due to both displacement currents in the circuitry and the plasma discharge itself. As such, sampling at the fundamental frequency provides little information about characteristics of the surface 14, as the signal is dominated by the stronger undistorted RF signal. By capturing a narrower bandwidth signal centred around harmonic frequencies, discharge related information can be extracted without having to extract much smaller plasma related signals from a large background.

Optionally, as is shown in Figure 1, the signal is passed through a filter 22, e.g. prior to the analysis. The filter may be a high pass filter with a pass band above *f₀* and below *2f₀,* or a notch filter centred around *f₀,* with a bandwidth larger than approximately 100 *fₘ*. Following filtering, the signal may then be linearly pre-amplified with an amplifier 24. The pre-amplified signal is then fed into an analyser 26. The analyser 26 may be a software-defined radio, or a spectrum analyser for example. In examples where the analyser is a software defined radio, the centre frequency of a local oscillator of the software defined radio may be set at *f₀*. The signal is then digitised and processed by performing a frequency domain analysis of the digitised signal (see S230 of Figure 2). In particular, the signal may be processed using a Fourier Transform algorithm, converting the time domain information into the frequency domain.

In some examples, in order to increase the number of features captured, multiple harmonic bands may be monitored and analysed, either simultaneously using a multi-channel RF front-end or by band hopping between successive harmonics.

Generally, only amplitude information may be preserved in this frequency domain analysis. In particular, the phase information of the signal may be removed. This may reduce the processing load, and therefore processing time, required to perform the analysis.

Optionally, the signal may be filtered to remove background noise. In some examples, background noise may be removed by sampling the background RF noise, and subtracting/removing the background signals from the signal obtained by antenna 20. For example, a secondary receiving antenna (not shown in Figure 1) may be positioned further away from the plasma discharge than antenna 20 (e.g. so as to not detect signals resulting from the plasma discharge impinging on the surface). This secondary receiving antenna may be used with an RF front-end to capture the background signals. The background signals captured by the secondary receiving antenna are treated in the same way as the plasma generated signals sampled by antenna 20. Peak detection is used on the background spectrum to identify interference and external noise (e.g. commercial communications) that may be present in the same band as the plasma harmonics (*nf₀*). Once identified, the interfering signal centre frequencies and bandwidths can be used to filter out the extraneous spectral peaks, using known digital signal processing methods. Alternatively, interference detection can be performed using antenna 20 before the plasma discharge is generated, to establish filter parameters and background noise without requiring a secondary antenna.

At S230 of method 200, frequency domain analysis of the signal is performed, e.g. to generate spectrograms 28 centred around the harmonics of the signal, *nf₀,* where n is an integer. Transforming the sampled RF signal into the frequency domain and discarding phase information allows for an improved signal-to-noise ratio by integration of successive RF samples.

Memory requirements for storing the signals are also decreased, as follows. The RF signal samples may be stored as a vector containing N bytes of time domain. By converting into the frequency domain, the data is converted into N/2 bytes of frequency domain data by exclusion of phase information. This may be further compressed via continuous averaging of p vectors per amplitude spectrogram, leading to a compression ratio of 1/2p. The averaging window size in turn determines the bandwidth of downstream analysis, with increasing p leading to slower analysis.

At S240 of method 200, an output signal indicative of a characteristic of the surface is generated based on the frequency domain analysis. The characteristic may be surface impedance, for example.

Multiple analysis methods for generated an output signal indicative of a characteristic of the surface from the obtained frequency domain spectrograms are possible. For example, the output signal may be generated, at least in part, by a machine learning process, including supervised and unsupervised methods. Unsupervised methods may be employed to differentiate between different features that might be present in the plasma exposed surfaces, such as electrical conductivity differences in tissues or sub-surface faults that might be present in otherwise conductive or insulating samples. Supervised learning methods, which may utilize deep neural networks, may be used with pre-collected training data for classification of different types of test surfaces by feeding in spectral features from labelled data and using the pre-trained neural network for classification of the surface characteristic.

The method may form part of a control process, where the output signal is fed back in a control loop to maintain a required (e.g. stable) plasma dissipated power. This provides improved control over the plasma discharge process, e.g. a plasma deposition process. As such, in S250 of method 200, the power delivered to the plasma discharge by an RF generator is adjusted based on the output signal indicative of the characteristic of the surface impedance. In particular, an applied voltage or current may be adjusted in order to adjust the power delivered. Alternatively/additionally, the frequency of the generator driving signal may be adjusted in order to adjust the dissipated power. In some examples, the power delivered by the RF generator may be adjusted such that the power dissipated to the surface is maintained as approximately constant.

The method may also comprise one or more surface characterisation steps e.g. to map the surface characteristics. At S260, a spatial and/or a temporal map of the characteristic of the surface may be generated by recording the output signal as a function of time and/or spatial position, respectively. A spatial map of the characteristic shows the changes in the characteristic over the surface. A temporal map shows changes in the characteristic over time. In order to generate a spatial map of the characteristic of the surface, the substrate 14 may be moved relative to the plasma nozzle (e.g. so that the plasma discharge impinges on a different portion of the substrate 14 as the substrate 14 is moved). The surface may be moved in 3D space (e.g. X-Y-Z directions).

To demonstrate the benefits of method 200, two example implementations of the method were carried out, and are described below.

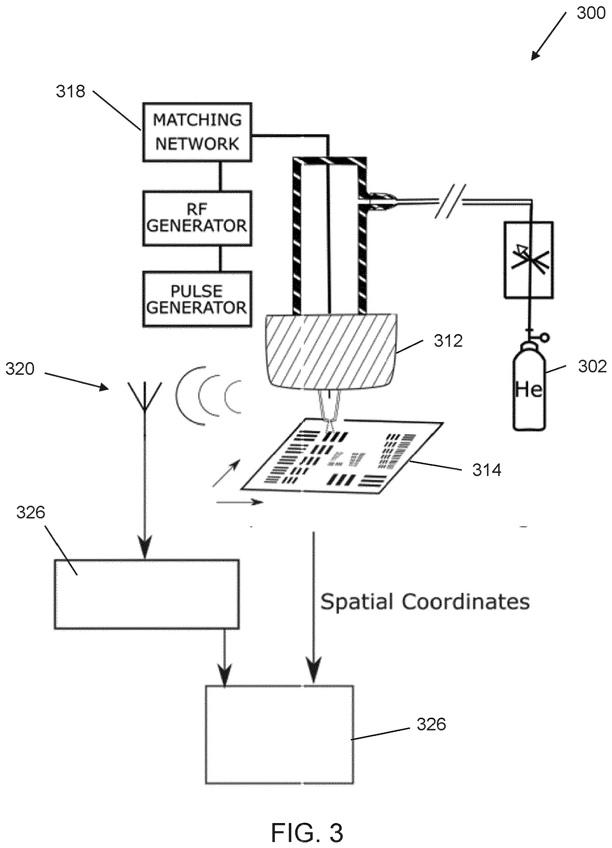

A schematic of system 300 of a first example implementation is shown in Figure 3. In the system 300, a micro plasma discharge was generated by a plasma nozzle 312 at atmospheric pressure using helium gas stored in canister 302. The plasma nozzle was used to scan the surface of a fibre reinforced resin circuit board 314 containing electrically floating metallic sections embedded under a continuous layer of polymer solder-mask that is 25 µm thick. This sample surface serves as an example of an electrically heterogeneous surface at radio frequency with an entirely DC insulating surface.

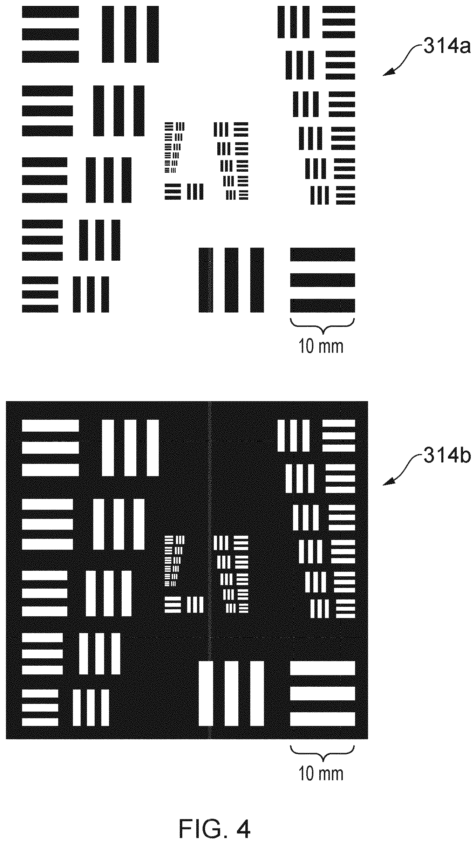

Figure 4 shows a test standard 314a of the reinforced circuit board 314 showing the positions of the conductive spots, and a physical sample 314b of the reinforced circuit board 314 with a clear solder-mask to show the floating copper conductors. As shown in Figure 4, the length of the conductors is approximately 10 mm.

This reinforced resin circuit 314 was used as a representative analogue of a surface with varying impedance, wherein the metallic patch determines the capacitance of the embedded metal layer. Conventionally, the detection of the metal section is not possible without damaging either the polymeric layer or by using tools such as X-ray imaging. However, surface mapping of the impedance/capacitance may be determined using the above described non-contact method for characterising a surface.

In particular, the resin circuit board 314 was placed on an X-Y translation stage while the plasma apparatus, including nozzle 312 is suspended above. Helium gas was fed from the canister 302 through a capillary (which, in this example implantation, had a 250 µm diameter orifice) and the nozzle 312 was configured to impinge a plume of helium gas on the circuit board 314 at a perpendicular angle relative to the main plane of the circuit board. A plasma electrode was placed within the helium capillary, and fed via a pulsed RF generator, through an impedance matching network 318, to generate a plasma discharge that was impinged on the circuit board 314.

During operation, the circuit board 314 was moved in a zig-zag pattern underneath the plasma discharge ejected from the capillary (as illustrated by the arrows shown in Figure 3). The plasma discharge was formed between a surface of the polymer layer of the circuit board 314, and the electrode tip, with a diameter approximately equal to the diameter of the orifice. Plasma current was passed through the plasma discharge, coupled to free space, and also to the circuit board 314. The presence of the embedded copper areas in the circuit board 314 between the plasma discharge and ground resulted in an increase in the capacitance of the effective circuit, leading to a drop in overall impedance of the plasma apparatus, even when the plasma discharge was not in direct contact with the copper layer. This led to a slightly perturbed modulation envelope.

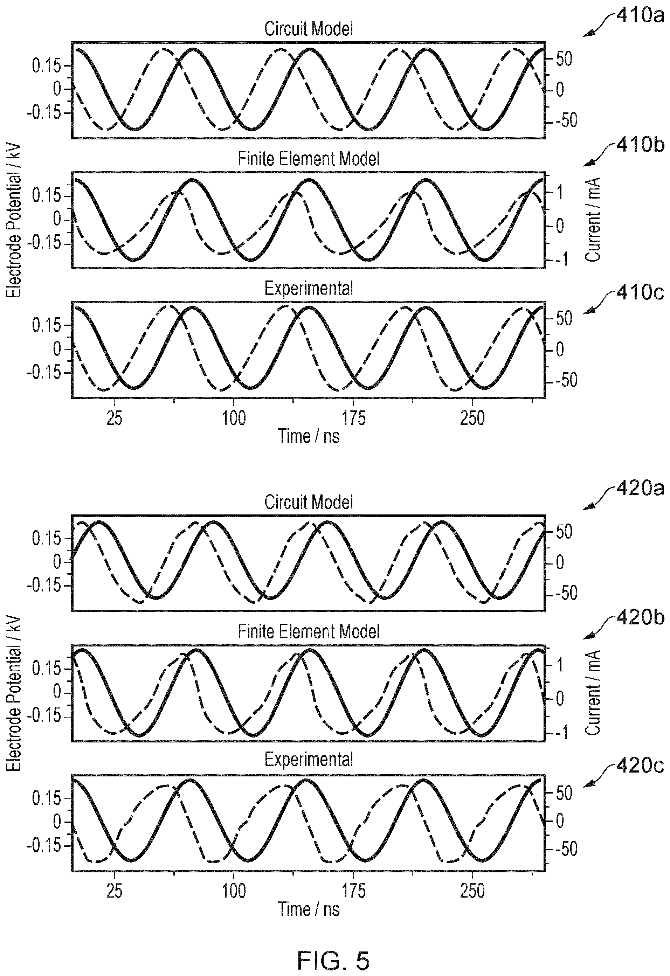

In particular, Figure 5 shows graphs of current and voltage waveforms as obtained experimentally and through simulations. Graphs 410a-c show the equivalent distortion observed when the plasma discharge is in contact with a dielectric where the simulated resistance is 10 MOhm and the dielectric constant is 4.

Graphs 410a and 410b are obtained by simulation and graph 410c is obtained by experiment. Graphs 420a-c show the distortion observed when the plasma discharge is in direct contact with RF ground. Graphs 420a and 420b are obtained by simulation and graph 420c is obtained by experiment. As shown by comparing graphs 410a-c and 420a-c, distortion in the waveform indicating current is visible.

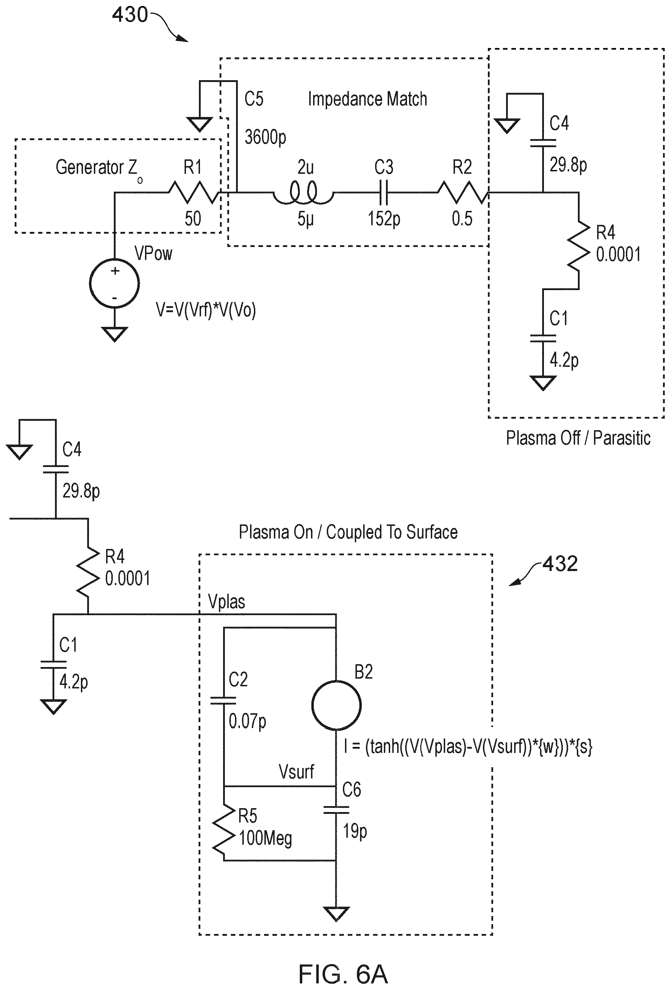

Figure 6A shows circuit diagrams 430, 432 associated with the plasma system shown in Figure 3. In particular, circuit diagram 430 is a circuit model of the RF source, matching network and the approximate plasma load without a substrate underneath. Circuit diagram 432 is a simulated plasma load in contact with a dielectric surface.

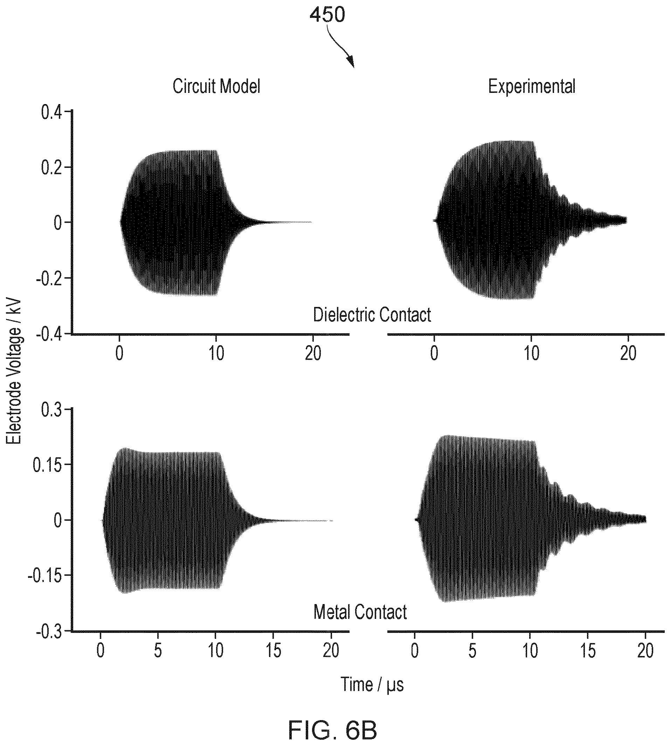

Figure 6B is a plot 450 of the resulting pulse envelopes under a 20 kHz / 20% duty cycle when the plasma discharge is in contact with a metallic contact and a dielectric contact. As shown in Figure 6B, a substantial change in the electrode voltage envelope is visible between the plots for the metallic contact and the dielectric contact, due to increased resistive loading on the plasma discharge.

By collecting the RF emission (signal) from the plasma discharge, the positions on the circuit board 314 where a metallic inclusion is present, could be deduced.

In this example implementation, the 6^{th} harmonic (81.36 MHz) of the carrier frequency (13.56 MHz) was chosen as the centre frequency for the RF signal acquisition. As shown in Figure 3, a software defined radio was used as the RF Front End analyser 326, with a 20 cm end fed antenna placed approximately 40 cm away from the electrode top to collect the signal. The antenna was followed by a 20 MHz high-pass filter that provided at least -2 dB of attenuation at the carrier frequency of 13.56 MHz.

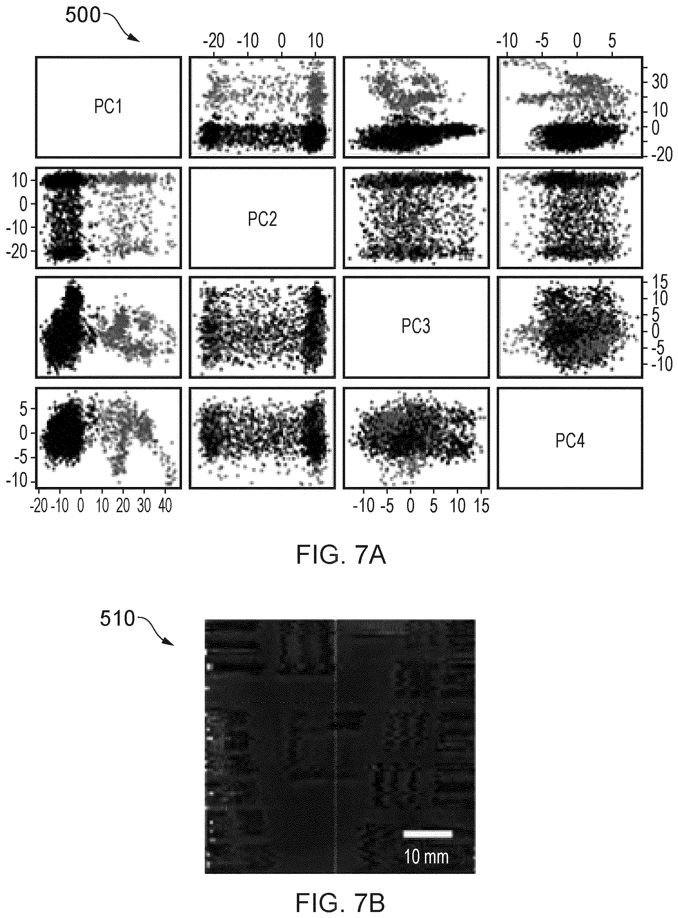

Known libraries (including Gnuradio C++ and Python) were then used for signal processing at analyser 326 (which may be a computer, for example). In particular, at each X-Y position (defined by spatial coordinates), RF waveforms were sampled for 100 ms, and translated into the frequency domain via Fast Fourier Transform (FFT). The resulting FFT spectrograms, which consisted of 1024 bins, were averaged until the substrate position was changed. For each point on the surface (i.e. each X-Y position at which the signal was sampled), a single spectrogram was saved in a binary format for post-processing. Unsupervised learning approaches were then used to obtain a surface map of the hidden metallic features. This was achieved using principal component analysis, followed by k-means clustering. Figure 7A shows pairwise plots 500 of principal component loadings for each datapoint in a map, with the three different shades indicating the binary clustering result. In particular, Figure 7A shows the three obtained clusters as plotted among dimensions of 4 principal components.

In different applications, the number of clusters can be chosen based on the number of plasma-surface interaction states that are expected. In the example implementation of circuit board 34, the two states of importance were the higher impedance state where the plasma discharge impinged on the bare PCB substrate, and the lower impedance state where capacitive coupling to a piece of conductor underneath the polymer coating is present.

By identifying one of the clusters as the high impedance state through a single "background" spectrogram, a spatial map 510 of the surface impedance at the harmonic of choice was generated for the circuit board 314, as shown in Figure 7B. The resolution of the map 510 was limited by the step size of the linear translation stage. As shown in spatial map 510 of Figure 7B, the location and orientation of the metallic sections embedded under the continuous layer of polymer solder-mask in the circuit board 314 can be found.

For completeness, a similar process can also be used with supervised machine learning approaches, where classified training data is collected for surfaces of interest before classification (or regression). An alternative method to principle component analysis may involve deep neural networks with normalised power spectrograms used as the input layer and classifier outputs.

A second example implementation was also carried out to demonstrate how the method described herein can be used as part of a process control loop, where certain parameters associated with the extracted spectrograms can be used to generate an "error" signal which can provide feedback. This feedback may be useful in the case, of surface treatment for example. In particular, as described hereafter, a closed loop power control of a plasma discharge deposition system may be provided.

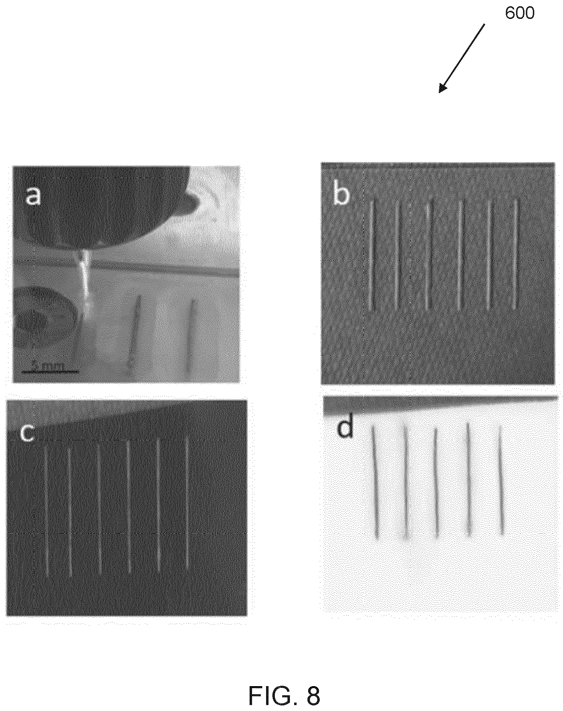

During this second example implementation, a set of apparatus as shown in Figure 3 was used, but with added metallic copper salts (copper II formate) in the gas supply in an aerosolised form. These added metallic copper salts can generate metallic tracks on the substrate whilst simultaneously sintering with the plasma discharge (as shown in images 600 of Figure 8).

During this process, a copper surface is generated on top of an insulting substrate. As explained above, this copper surface is then electrically coupled to the plasma discharge, causing a lower impedance path for current flow and thus simultaneously increasing the power dissipated on the substrate surface. If the dissipated power becomes too low, the deposition process stops or becomes less effective, leading to low-conductivity traces on the surface. Conversely, if the dissipated power is too high, polymeric substrates may be subjected to thermal damage. As this deposition process adds conductive layers to the surface, it is desirable to actively monitor the power coupled to the surface to maintain the dissipated power within predefined acceptable limits. As mentioned above, conventionally there were no simple methods of deducing the amount of power dissipated at the interface between the plasma discharge and the target substrate. Optical spectroscopy was one of the few methods to implement such a control feature. However, the method described above allows for the amount of power dissipated at the surface interface to be deduced non-invasively.

In this example implementation, the substrate was moved at a steady 1 mm/s rate under the plasma discharge (shown in Figure 3) whilst a track of conductive copper was deposited onto the surface. Prior to deposition, reference data for the plasma discharge in direct contact with a metallic surface and a plastic surface was collected and processed by the analyser (as described above with respect to the first example implementation), and the principal components along which the two states are most distinct were identified.

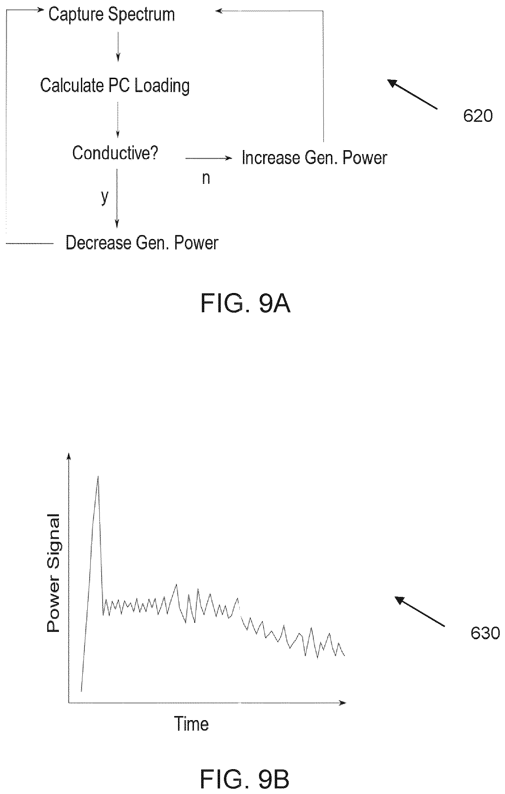

In an ideal case, the deposition should proceed at the lowest power dissipation possible without resulting in non-conductive deposits. This was achieved by decreasing the power control signal by a predefined amount, ΔP-, each time the obtained sample was classified as conductive, and increasing the power control signal by the predefined amount, ΔP+, when an insulating surface was detected. A decision tree 620 showing this logic is shown in Figure 9A.

This resulted in steady oscillation in the power level as the system aims for optimal output level. In the present example implementation, as the deposited metal area increases, the optimal output power level is not steady because the plasma discharge impedance decreases accordingly, requiring decreased generator power to achieve similar deposition quality. Figure 9B shows a plot 630 of the power signal used to control RF power amplifier levels during a deposition process. As shown in plot 630, the power signal oscillates between two levels and steadily drifts towards a lower power output as the amount (e.g. length) of the conductive surface is increased.

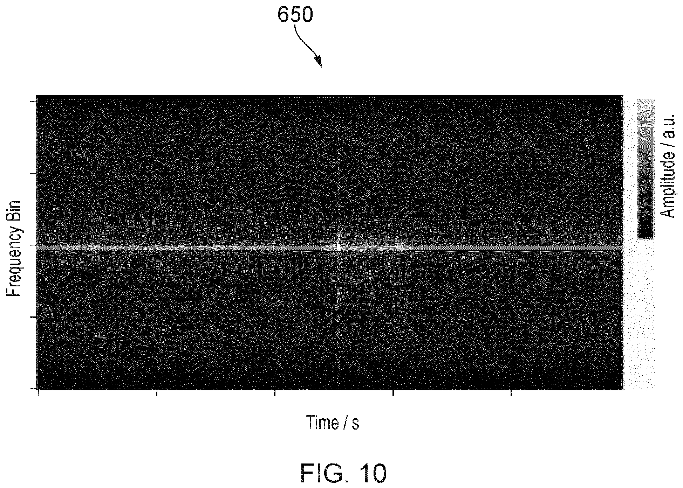

To increase the reliability of the power feedback look, an error signal may be generated by calculating a cross correlation between a sample and the desired operating spectrogram, or radial distance between a centroid of the k-means cluster and an incoming sample position. This error signal may be used as part of a well-tuned proportional-integral-derivative (PID) loop to ensure that the oscillatory behaviour in the plasma control loop is minimized. Similarly, any samples that lie outside boundaries of a predefined feature space can be logged, and processed to ensure any process anomalies are accounted for. Figure 10 shows a waterfall plot 650 of a plasma discharge periodically encountering a grounded surface, where the changes in spectral features are apparent. At these potential faults, it may be possible to log the fault condition, and stop generation of the plasma, if needed.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

The exemplary embodiments of the invention set forth above are considered to be illustrative and not limiting. Various changes to the described embodiments may be made without departing from the scope of the invention

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A method for monitoring a characteristic of a surface (14; 314) while an atmospheric-pressure dual-frequency plasma discharge is impinging on the surface, the method comprising:
receiving a signal emitted from the plasma discharge impinging on the surface;
performing a frequency domain analysis of the signal; and
generating an output signal indicative of a characteristic of the surface based on the frequency domain analysis.

2. The method of claim 1, wherein the signal is captured using an antenna (20; 320).

3. The method of claim 1 or claim 2, further comprising:
adjusting the power delivered to the plasma discharge by a generator based on the output signal, and optionally wherein the power delivered by the generator is adjusted such that the power dissipated to the surface is maintained as approximately constant.

4. The method of any preceding claim, further comprising:
generating a spatial and/or temporal map of the characteristic of the surface by recording the output signal as a function of time and/or spatial position, and optionally further comprising outputting the spatial/temporal map of the characteristic of the surface for display.

5. The method of any preceding claim, wherein the plasma discharge is impinging on the surface to deposit, remove or modify a layer thereon.

6. The method of any preceding claim, wherein the characteristic of the surface is the surface impedance.

7. The method of any preceding claim, wherein the frequency domain analysis comprises generating a power spectral density by performing a Fourier Transform analysis, and optionally wherein the output signal is generated at least in part by performing a data fitting process to resonant features of the power spectral density.

8. The method of any preceding claim, wherein the frequency domain analysis comprises:
evaluating resonant features of at least one higher harmonic of a driving signal present in the signal; and/or
evaluating a relative intensity of a plurality of higher harmonics of a driving signal present in the signal.

9. The method of any preceding claim, wherein the output signal is generated at least in part by a machine learning process.

10. The method of any preceding claim, further comprising:
filtering the signal to remove high power signals and/or to remove background noise; and/or
capturing, using an antenna, the signal emitted from the plasma discharge impinging on the surface.

11. The method of any preceding claim, wherein:
the plasma discharge is used in a process for plasma activating and/or cleaning the surface;
and/or
the plasma discharge is an RF plasma discharge.

12. A system (10; 300) for monitoring a characteristic of a surface (14; 314) while an atmospheric-pressure dual-frequency plasma discharge is impinging on the surface, the system comprising:
a plasma apparatus, the plasma apparatus comprising a radio frequency generator and an atmospheric-pressure plasma nozzle (12; 312) configured to be driven by the radio-frequency generator, wherein the plasma apparatus is configured to generate the atmospheric-pressure dual-frequency plasma discharge, and wherein the plasma nozzle is configured to discharge the atmospheric-pressure dual-frequency plasma discharge;
and
one or more processors configured to:
receive a signal emitted from the plasma discharge impinging on the surface;
perform a frequency domain analysis of the signal; and
generate an output signal indicative of a characteristic of the surface based on the frequency domain analysis.

13. The system of claim 12, further comprising an antenna (20; 320), wherein the antenna is configured to capture the signal emitted from the plasma discharge impinging on the surface.

14. Use of a system for monitoring a characteristic of a surface while an atmospheric-pressure dual-frequency plasma discharge is impinging on the surface, with a plasma apparatus comprising a radio frequency generator and an atmospheric-pressure plasma nozzle configured to be driven by the radio frequency generator, wherein the system comprises one or more processors configured to:
receive a signal emitted from the plasma discharge impinging on the surface;
perform a frequency domain analysis of the signal; and
generate an output signal indicative of a characteristic of the surface based on the frequency domain analysis.

15. A non-transitory computer readable storage medium containing machine executable instructions which, when executed on a processor, cause the processor to perform the method of any of claims 1-11.

## Patentansprüche

1. Verfahren zum Überwachen einer Eigenschaft einer Fläche (14, 314), während eine Atmosphärendruck-Doppelfrequenzplasmaentladung auf die Fläche auftrifft, wobei das Verfahren Folgendes umfasst:
Empfangen eines Signals, das von der auf die Fläche auftreffenden Plasmaentladung emittiert wird;
Durchführen einer Frequenzbereichsanalyse des Signals; und
Erzeugen eines Ausgangssignals, das eine Eigenschaft der Fläche angibt, beruhend auf der Frequenzbereichsanalyse.

2. Verfahren nach Anspruch 1, wobei das Signal unter Verwendung einer Antenne (20, 320) erfasst wird.

3. Verfahren nach Anspruch 1 oder 2, ferner umfassend:
Einstellen der Leistung, die von einem Generator für die Plasmaentladung zugeführt wird, beruhend auf dem Ausgangssignal, und wobei gegebenenfalls die von dem Generator zugeführte Leistung derart eingestellt wird, dass die zur Fläche abgeleitete Leistung annähernd konstant gehalten wird.

4. Verfahren nach einem der vorangegangenen Ansprüche, ferner Folgendes umfassend:
Erzeugen einer räumlichen und/oder zeitlichen Karte der Eigenschaft der Fläche durch Aufzeichnen des Ausgangssignals als Funktion von Zeit und/oder räumlicher Position, und ferner gegebenenfalls umfassend das Ausgeben der räumlichen/zeitlichen Karte der Eigenschaft der Fläche zum Anzeigen.

5. Verfahren nach einem der vorangegangenen Ansprüche, wobei die Plasmaentladung auf die Fläche auftrifft, um eine Schicht darauf aufzubringen, zu entfernen oder zu ändern.

6. Verfahren nach einem der vorangegangenen Ansprüche, wobei die Eigenschaft der Fläche die Oberflächenimpedanz ist.

7. Verfahren nach einem der vorangegangenen Ansprüche, wobei die Frequenzbereichsanalyse das Erzeugen einer Leistungsspektraldichte durch Durchführen einer Fourier-Transformations-Analyse umfasst und wobei gegebenenfalls das Ausgangssignal zumindest teilweise durch Durchführen eines Datenanpassungsvorgangs für Resonanzmerkmale der Leistungsspektraldichte erzeugt wird.

8. Verfahren nach einem der vorangegangenen Ansprüche, wobei die Frequenzbereichsanalyse Folgendes umfasst:
Bewerten von Resonanzmerkmalen von zumindest einer höheren Harmonischen eines Ansteuersignals, das in dem Signal vorliegt; und/oder
Bewerten einer relativen Intensität einer Vielzahl von höheren Harmonischen eines Ansteuersignals, das in dem Signal vorliegt.

9. Verfahren nach einem der vorangegangenen Ansprüche, wobei das Ausgangssignal zumindest teilweise durch einen Maschinelles-Lernen-Vorgang erzeugt wird.

10. Verfahren nach einem der vorangegangenen Ansprüche, ferner umfassend:
Filtern des Signals, um Hochleistungssignale zu entfernen und/oder Hintergrundrauschen zu entfernen; und/oder
Erfassen des Signals, das von der auf der Fläche auftreffenden Plasmaentladung erzeugt wurde, unter Verwendung einer Antenne.

11. Verfahren nach einem der vorangegangenen Ansprüche, wobei:
die Plasmaentladung in einem Vorgang zur Plasmaaktivierung und/oder Reinigung der Fläche verwendet wird; und/oder
die Plasmaentladung eine HF-Plasmaentladung ist.

12. System (10, 300) zum Überwachen einer Eigenschaft einer Fläche (14, 314), während eine Atmosphärendruck-Doppelfrequenzplasmaentladung auf die Fläche auftrifft, wobei das System Folgendes umfasst:
eine Plasmavorrichtung, wobei die Plasmavorrichtung einen Hochfrequenzgenerator und eine Atmosphärendruckplasmadüse (12, 312) umfasst, die dazu ausgelegt ist, von dem Hochfrequenzgenerator angesteuert zu werden,
wobei die Plasmavorrichtung dazu ausgelegt ist, die Atmosphärendruck-Doppelfrequenzplasmaentladung zu erzeugen und wobei die Plasmadüse dazu ausgelegt ist, die Atmosphärendruck-Doppelfrequenzplasmaentladung zu entladen; und
einen oder mehrere Prozessoren, die für Folgendes ausgelegt sind:
Empfangen eines Signals, das von der auf die Fläche auftreffenden Plasmaentladung emittiert wird;
Durchführen einer Frequenzbereichsanalyse des Signals; und
Erzeugen eines Ausgangssignals, das eine Eigenschaft der Fläche angibt, beruhend auf der Frequenzbereichsanalyse.

13. System nach Anspruch 12, ferner umfassend eine Antenne (20, 320), wobei die Antenne dazu ausgelegt ist, das von der auf der Fläche auftreffenden Plasmaentladung emittierte Signal zu erfassen.

14. Verwendung eines Systems zum Überwachen einer Eigenschaft einer Fläche, während eine Atmosphärendruck-Doppelfrequenzplasmaentladung auf die Fläche auftrifft, mit einer Plasmavorrichtung, die einen Hochfrequenzgenerator und eine Atmosphärendruckplasmadüse umfasst, die dazu ausgelegt ist, von dem Hochfrequenzgenerator angesteuert zu werden, wobei das System einen oder mehrere Prozessoren umfasst, die für Folgendes ausgelegt sind:
Empfangen eines Signals, das von der auf die Fläche auftreffenden Plasmaentladung emittiert wird;
Durchführen einer Frequenzbereichsanalyse des Signals; und
Erzeugen eines Ausgangssignals, das eine Eigenschaft der Fläche angibt, beruhend auf der Frequenzbereichsanalyse.

15. Nichtflüchtiges computerlesbares Speichermedium, das durch eine Maschine ausführbare Befehle enthält, welche, wenn sie auf einem Prozessor ausgeführt werden, bewirken, dass der Prozessor ein Verfahren nach einem der Ansprüche 1 bis 11 durchführt.

## Revendications

1. Procédé de surveillance d'une caractéristique d'une surface (14 ; 314) pendant qu'une décharge de plasma à double fréquence à pression atmosphérique heurte la surface, le procédé comprenant les étapes consistant à :
recevoir un signal émis à partir de la décharge de plasma heurtant la surface ;
effectuer une analyse de domaine de fréquence du signal ; et
générer un signal de sortie indicatif d'une caractéristique de la surface sur la base de l'analyse de domaine de fréquence.

2. Procédé selon la revendication 1, dans lequel le signal est capturé en utilisant une antenne (20 ; 320).

3. Procédé selon la revendication 1 ou la revendication 2, comprenant en outre l'étape consistant à :
ajuster la puissance délivrée à la décharge de plasma par un générateur sur la base du signal de sortie, et facultativement dans lequel la puissance délivrée par le générateur est ajustée de telle sorte que la puissance dissipée vers la surface soit maintenue comme étant approximativement constante.

4. Procédé selon l'une quelconque des revendications précédentes, comprenant en outre l'étape consistant à :
générer une carte spatiale et/ou temporelle de la caractéristique de la surface en enregistrant le signal de sortie en fonction du temps et/ou de la position spatiale, et comprenant en outre facultativement l'étape consistant à délivrer en sortie la carte spatiale/temporelle de la caractéristique de la surface pour affichage.

5. Procédé selon l'une quelconque des revendications précédentes, dans lequel la décharge de plasma heurte la surface pour déposer, enlever ou modifier une couche sur celle-ci.

6. Procédé selon l'une quelconque des revendications précédentes, dans lequel la caractéristique de la surface est l'impédance de surface.

7. Procédé selon l'une quelconque des revendications précédentes, dans lequel l'analyse de domaine de fréquence comprend l'étape consistant à générer une densité spectrale de puissance en effectuant une analyse par transformée de Fourier, et facultativement dans lequel le signal de sortie est généré au moins en partie en effectuant un processus d'ajustement de données par rapport à des caractéristiques résonantes de la densité spectrale de puissance.

8. Procédé selon l'une quelconque des revendications précédentes, dans lequel l'analyse de domaine de fréquence comprend les étapes consistant à :
évaluer des caractéristiques résonnantes d'au moins une harmonique supérieure d'un signal d'entraînement présent dans le signal ; et/ou
évaluer une intensité relative d'une pluralité d'harmoniques supérieures d'un signal d'entraînement présent dans le signal.

9. Procédé selon l'une quelconque des revendications précédentes, dans lequel le signal de sortie est généré au moins en partie par un processus d'apprentissage machine.

10. Procédé selon l'une quelconque des revendications précédentes, comprenant en outre les étapes consistant à :
filtrer le signal pour éliminer les signaux de forte puissance et/ou pour éliminer le bruit de fond ; et/ou
capturer, en utilisant une antenne, le signal émis par la décharge de plasma heurtant la surface.

11. Procédé selon l'une quelconque des revendications précédentes, dans lequel :
la décharge de plasma est utilisée dans un processus d'activation et/ou de nettoyage de la surface par plasma ; et/ou
la décharge de plasma est une décharge de plasma RF.

12. Système (10 ; 300) pour surveiller une caractéristique d'une surface (14 ; 314) alors qu'une décharge de plasma à double fréquence à pression atmosphérique heurte la surface, le système comprenant :
un appareil à plasma, l'appareil à plasma comprenant un générateur de radiofréquence et une buse à plasma à pression atmosphérique (12 ; 312) configurée pour être entraînée par le générateur de radiofréquence, dans lequel l'appareil à plasma est configuré pour générer la décharge de plasma à double fréquence à pression atmosphérique, et dans lequel la buse à plasma est configurée pour décharger la décharge de plasma à double fréquence à pression atmosphérique ; et
un ou plusieurs processeurs configurés pour :
recevoir un signal émis à partir de la décharge de plasma heurtant la surface ;
effectuer une analyse de domaine de fréquence du signal ; et
générer un signal de sortie indicatif d'une caractéristique de la surface sur la base de l'analyse de domaine de fréquence.

13. Système selon la revendication 12, comprenant en outre une antenne (20 ; 320), dans lequel l'antenne est configurée pour capturer le signal émis par la décharge de plasma heurtant la surface.

14. Utilisation d'un système pour surveiller une caractéristique d'une surface alors qu'une décharge de plasma à double fréquence à pression atmosphérique heurte la surface, avec un appareil à plasma comprenant un générateur de radiofréquence et une buse à plasma à pression atmosphérique configurée pour être entraînée par le générateur de radiofréquence, dans lequel le système comprend un ou plusieurs processeurs configurés pour :
recevoir un signal émis à partir de la décharge de plasma heurtant la surface ;
effectuer une analyse de domaine de fréquence du signal ; et
générer un signal de sortie indicatif d'une caractéristique de la surface sur la base de l'analyse de domaine de fréquence.

15. Support de stockage non transitoire lisible par ordinateur contenant des instructions exécutables par machine qui, lorsqu'elles sont exécutées sur un processeur, amènent le processeur à exécuter le procédé selon l'une quelconque des revendications 1 à 11.
